# EUROPEAN PATENT SPECIFICATION

(11) **EP 1 047 469 B1**
(45) Date of publication and mention of the grant of the patent: **18.06.2003**
(21) Application number: 99903982.9
(22) Date of filing: 15.01.1999
(51) Int. Cl.: A61M 16/04, A61M 25/02

(54) **A DEVICE FOR THE FIXATION OF A TUBE MEMBER**
VORRICHTUNG ZUM BEFESTIGEN EINES SCHLAUCHELEMENTES
DISPOSITIF POUR LA FIXATION D'UN ELEMENT TUBULAIRE

(30) Priority: 15.01.1998 SE 9800078
(43) Date of publication of application: 02.11.2000
(73) Proprietor: Cardia Innovation AB, 114 32 Stockholm (SE)
(72) Inventor: VAN DER LINDEN, Jan, S-133 34 Saltsjöbaden (SE)
(74) Representative: Berglund, Stefan
(86) International application number: SE9900052
(87) International publication number: WO99036117

(56) References cited:
- WO-A1-95/18645
- US-A- 5 221 265
- US-A- 5 546 938

## Description

### THE BACKGROUND OF THE INVENTION AND PRIOR ART

The present invention refers to a device for the fixation of a tube member to be introduced into an opening in the body of a patient, comprising a foil-shaped piece of material with a first side surface and an opposite second side surface, which is provided with an adhesive, wherein the piece of material comprises a base portion.

A device as defined in the preamble of claim 1 is disclosed in WO-A-95/18645.

The invention in this application is described in relation to the fixation of oropharyngeal tubes or so-called endotracheal tubes, i.e. tubes which are normally produced from a plastic material or a rubber material, and which are inserted through the mouth or nose and into the trachea of a patient for the supply of air. Such endotracheal tubes are used for so-called intubation in connection with anaesthetic, and possibly the air supplied is mixed with an anaesthetic gas for anaesthetization of the patient. However, the device according to the invention is not exclusively applicable to this use of endotracheal tubes but may also be used in connection with other therapeutic or diagnostic methods.

Today, common tape of surgery is utilised for such fixation of endotracheal tubes, which tape is wound several rounds around the endotracheal tube and which is attached to the mouth of the patient in the best possible way for the occasion. This method is very difficult to carry out in an elegant way and is also time-consuming, which of course can be a very severe drawback in relation to an acute operation.

Moreover, it is by this known method difficult to fixate the endotracheal tube in a safe and reliable manner in the desired position. With an incorrect fixation, the endotracheal tube can easily take a wrong position beside the trachea which may result in an insufficient quantity of breathing air to the anaesthetised patient.

### SUMMARY OF THE INVENTION

The object of the present invention is to overcome the problems mentioned above and in particular to provide a device, which simplifies the fixation of a tube member in an opening of a patient. The invention also refers to a device by which this fixation becomes safer and more reliable than with previously used techniques.

This object is obtained by the device as defined in claim 1. By such a device it is possible to achieve a simple and reliable fixation of, for example, an endotracheal tube so that it extends through the mouth or the nose of a patient. Thereby, one of said leg portions may be used for attaching the piece of material to the endotracheal tube while the remaining portions may be used for the application to the skin of the patient. In particular, the base portion can be provided to be applied to the skin of the patient with the aid of said adhesive. The base portion, which can be made in an arbitrary size, will in this way guarantee the fixation of the device on the patient. Furthermore, the first leg portion may advantageously be provided to be attached to the skin on one side of said opening of the patient, the second intermediate leg portion may be provided to be applied to said tube member and the third leg portion may be provided to be attached to the skin on the other side of said opening of the patient.

According to a preferred embodiment of the invention, at least one of the three leg portions has an elongated shape.

According to a further embodiment of the invention, the intermediate leg portion is longer than the first and third leg portions. Thereby, it is achieved that the intermediate leg portion may be safely secured around, for example, an endotracheal tube.

According to the invention, a protective foil is detachably arranged to the second side surface of the piece of material and is provided to be removed before the device is applied to said patient. Such a protective foil can for example be produced from paper or plastic. According to the invention, the protective foil comprises four separate foil portions with a shape which covers at least the second side surface of each of said base and leg portions. In such a way, it is possible to remove the protective foil from the different portions, as they are to be applied to the skin and said tube member, respectively. Moreover, each protective foil portion may comprise a flap which projects in a direction away from the second side surface of each of said base and leg portions. Such a flap can in a simple manner be grasped by the person who shall apply the device and thereby the application of the device is further facilitated.

According to a further embodiment of the invention, the second side surface has a corner area of at least one of said base and leg portions, which is not provided with said adhesive. By such a limited area which is free from adhesive and which is situated in the vicinity of the edge of at least one of said portions, it is possible to remove the piece of material in a simple manner from the skin of the patient and from said tube member.

### BRIEF DESCRIPTION OF THE DRAWINGS

The present invention will now be explained more closely by a description of different embodiments and with reference to the attached drawings.
- Fig 1: discloses a perspective view of a device according to a first embodiment of the present invention.
- Fig 2: discloses a view from above of the device in Fig 1.
- Fig 3: discloses a view from below of the device in Fig 1.
- Fig 4: discloses a sideview of the device in Fig 1.
- Fig 5: discloses a perspective view of a device according to a second embodiment of the present invention.
- Fig 6: discloses a use of a device according to the present invention.

### DETAILED DESCRIPTION OF DIFFERENT EMBODIMENTS OF THE INVENTION

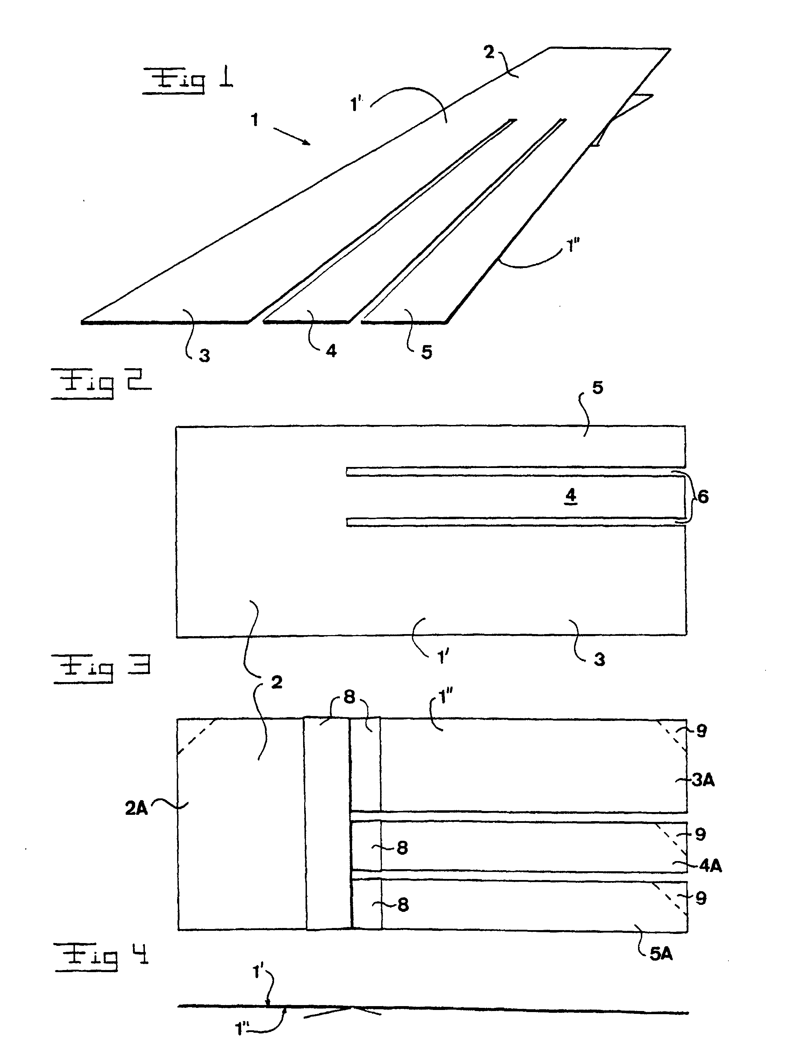

With reference to Fig 1 - 4, a device for the fixation of a tube member to be introduced into an opening in the body of the patient is shown. Such a tube member may have a diameter, which is between 1 mm and 40 mm. The device is henceforth described in connection with endotracheal tubes to be introduced into the mouth of the patient. However, it is possible within the scope of the invention to insert the endotracheal tube through the nose and fixate it with the aid of the device according to the invention. The device comprises a foil-shaped piece of material 1 with a first side surface 1' and an opposite second side surface 1''. On the second side surface 1'' an adhesive is provided. This adhesive can be of a conventional type, i.e. such adhesives which is today used for plaster, tape of surgery and for other medical applications, where something is to be attached to the skin of a patient. The piece of material 1 may also be produced from a number of different today available materials for plaster and tapes, which are used to be attached to the skin. For example, the piece of material 1 can be produced from a thin transparent plastic foil, textile, for example silk, paper and other synthetic materials.

The piece of material 1 comprises a base portion 2, a first leg portion 3, a second leg portion 4 and a third leg portion 5. The first, second and third leg portions 3, 4, 5 are separated from each other with a thin gap 6 between the first outer leg portion 3 and the second intermediate leg portion 4 as well as between the second intermediate leg portion 4 and the third outer leg portion 5. The three leg portions 3, 4, 5 extend from the base portion 1 substantially in parallel to each other in substantially the same direction. Such as shown in Fig 1-3, all the three leg portions 3, 4, 5 have an elongated shape. The total length of the piece of material 1 can be about 15-25 cm, for example about 21 cm, wherein the length of the base portion 2 is about 7 cm and the length of the three leg portions 3, 4, 5 are about 14 cm. The width of the piece of material 1 can be between about 5 and 10 cm, for example 6 cm, wherein the width of the first leg portion 3 can be about 3 cm and the width of each of the two remaining leg portions 4 and 5 are about 1.5 cm. The exemplified dimension is suitable for the application of an endotracheal tube into the mouth of an adult person while the piece of material 1 can have a smaller dimension for application of an endotracheal tube to children.

Furthermore, the device comprises a protective cover which is detachably arranged on the second side surface 1'' of the piece of material 1 to protect the adhesive. Before the device is applied to a patient the protective foil is removed, wherein the adhesive is uncovered. The protective foil comprises four separated foil portions 2A, 3A, 4A, 5A, which have a shape that covers the second side surface 1'' of each of the base and leg portions 2-5. Each foil portions 2A-5A is bent around itself to form a flap 8, which projects away from the second side surface 1'' in the border area between the base portion 2 and the three leg portions 3, 4, 5.

The second side surface 1'' is not provided with any adhesive in a corner area 9 of the base portion 2 and the three leg portions 3, 4, 5 in order to facilitate the removal of the device from a patient.

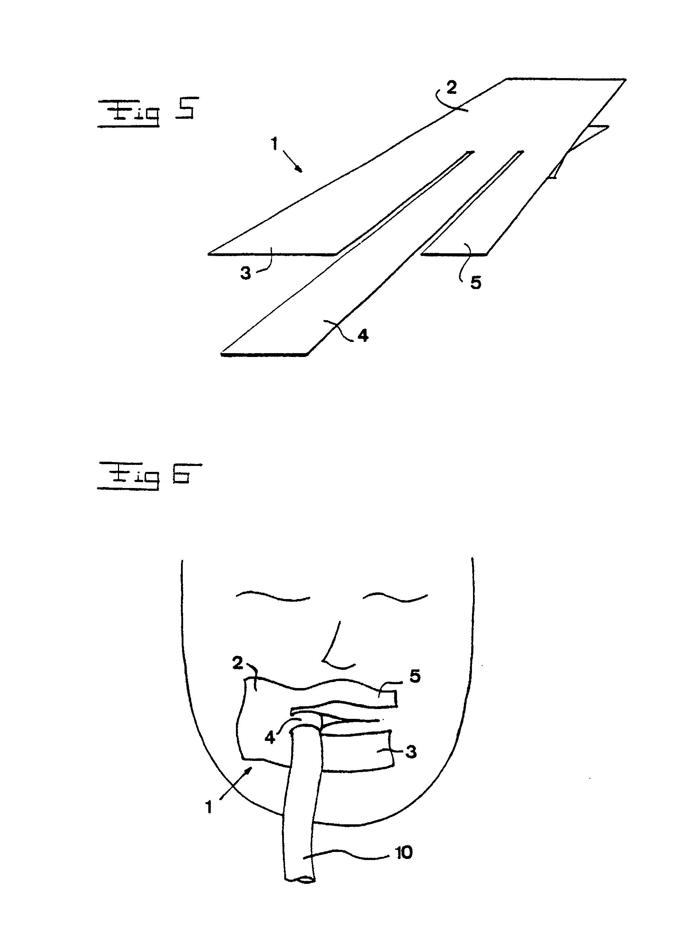

Fig 5 shows a second embodiment of the device according to the invention. According to this embodiment, the intermediate second leg portion 4 is longer than the two outer leg portions 3 and 5. Otherwise, the two embodiments are substantially equal. The second intermediate leg portion 4 may thereby have a length between 5 and 15 cm, for example 10 cm.

In fig 6, the device according to the invention is shown in a schematical way in a possible use. Thereby, the base portion 2 of the piece of material 1 is applied to the cheek of a patient while the first leg portion 3 and the third leg portion 5 are applied to the skin below and above the mouth of the patient. The second intermediate portion 4 is attached to the endotracheal tube 10. Thereby, the second intermediate leg portion 4 may be wound around the endotracheal tube 10, possibly by several rounds at least in case the second leg portion 4 is longer than the first and third leg portions 3, 5.

The present invention is not limited to the embodiments shown above but may be varied and modified within the scope of the following patent claims.

## Claims

1. A device for the fixation of a tube member (10) to be introduced into an opening in the body of a patient, comprising a foil-shaped piece of material (1) with a first side surface (1') and an opposite second side surface (1''), which is provided with an adhesive, wherein the piece of material (1) comprises a base portion (2), a first leg portion (3), a second leg portion (4) and a third leg portion (5), which are separated from each other and extend from the base portion substantially in parallel to each other in substantially the same direction, **characterized in that** four separate protective foil portions (2A - 5A) are detachably arranged to the second side surface (1'') of the piece of material (1) to cover each of said base and leg portions (2 - 5), respectively, wherein the foil portions are provided to be removed before the device is applied to said patient.

2. A device according to claim 1, **characterized in that** the base portion (2) is provided to be applied to the skin of said patient with the aid of said adhesive.

3. A device according to claim 2, **characterized in that** the first leg portion (3) is provided to be attached to the skin on one side of said opening of the patient, that the second intermediate leg portion (4) is provided to be attached to said tube member (10) and that the third leg portion (5) is provided to be attached to the skin on the other side of said opening of the patient.

4. A device according to any one of the preceding claims, **characterized in that** at least one of the three leg portions (3 - 5) has an elongated shape.

5. A device according to any one of the preceding claims, **characterized in that** the second intermediate leg portion (4) is longer than the first and third leg portions (3, 5).

6. A device according to any one of the preceeding claims, **characterized in that** each of the foil portions (2A - 5A) comprises a flap (8) which projects in a direction away from the second side surface (1'') of each of said base and leg portions (2 - 5).

7. A device according to any one of the preceding claims, **characterized in that** the second side surface (1'') has a corner area (9) of at least one of said base and leg portions (2 - 5), which is not provided with said adhesive.

8. A device according to any one of the preceding claims, **characterized in that** said tube member comprises a hose-like tube, which is produced from a plastic or rubber material.

9. A device according to any one of the preceding claims, **characterized in that** said tube member comprises an endotracheal tube.

## Patentansprüche

1. Vorrichtung zur Befestigung eines Schlauchelementes (10), das in eine Körperöffnung eines Patienten eingeführt werden soll, bestehend aus einem folienartigen Materialstück (1) mit einer ersten Seitenoberfläche (1') und einer gegenüberliegenden Seitenoberfläche (1"), die mit einem Klebstoff versehen ist, wobei das Materialstück (1) einen Basisteil (2), ein erstes Bein-Teil (3), ein zweites Bein-Teil (4) und ein drittes Bein-Teil (5) besitzt, die jeweils voneinander getrennt sind und sich von dem Basisteil im wesentlichen parallel zueinander in einer im wesentlichen gleichen Richtung erstrecken,
**dadurch gekennzeichnet,**
**dass** vier separate Schutzfolienteile (2A - 5A) lösbar zur Abdeckung des Basisteiles und jedes Beinteiles (2 bis 5) auf der zweiten Seitenoberfläche (1") des Materialstückes (1) angeordnet sind bzw. die Folienteile zur Entfernung vorgesehen sind, bevor die Vorrichtung beim Patienten angelegt wird.

2. Vorrichtung nach Anspruch 1, **dadurch gekennzeichnet, dass** das Basisteil (2) zur Anlage an der Haut des Patienten mit Hilfe des genannten Klebers vorgesehen ist.

3. Vorrichtung nach Anspruch 2, **dadurch gekennzeichnet, dass** das erste Bein-Teil (3) zur Befestigung auf der Haut auf einer Seite der genannten Patienten-öffnung vorgesehen ist, dass das zweite Zwischenbein-Teil (4) zur Befestigung an dem genannten Schlauchelement vorgesehen ist, und dass das dritte Bein-Teil (5) zur Befestigung an der Haut auf der anderen Seite der Patientenöffnung vorgesehen ist.

4. Vorrichtung nach einem der vorhergehenden Ansprüche, **dadurch gekennzeichnet, dass** wenigstens eins der drei Bein-Teile (3 bis 5) eine längliche Form hat.

5. Vorrichtung nach einem der vorhergehenden Ansprüche, **dadurch gekennzeichnet, dass** das zweite Zwischenbein-Teil (4) länger als das erste und das dritte Bein-Teil (3, 5) ist.

6. Vorrichtung nach einem der vorhergehenden Ansprüche, **dadurch gekennzeichnet, dass** jedes der Folienteile (2A - 5A) eine Lasche (8) besitzt, die in einer Richtung vorsteht, die von der zweiten Seitenoberfläche (1") des Basisteils sowie der Bein-Teile (2 bis 5) weggerichtet ist.

7. Vorrichtung nach einem der vorhergehenden Ansprüche, **dadurch gekennzeichnet, dass** die zweite Seitenoberfläche (1") eine Eckenfläche (9) wenigstens auf dem Basisteil oder einem der Bein-Teile (2 bis 5) besitzt, die nicht mit einem Klebstoff versehen ist.

8. Vorrichtung nach einem der vorhergehenden Ansprüche, **dadurch gekennzeichnet, dass** das genannte Schlauchelement ein Schlauchstück ist, das aus Kunststoff oder Gummimaterial hergestellt ist.

9. Vorrichtung nach einem der vorhergehenden Ansprüche, **dadurch gekennzeichnet, dass** das Schlauchelement ein endotrachealer Schlauch ist.

## Revendications

1. Dispositif pour la fixation d'un élément en forme de tube (10) à introduire dans une ouverture dans le corps d'un patient, comprenant un morceau de matière en forme de feuille (1) avec une première surface latérale (1') et une seconde surface latérale opposée (1"), qui est pourvue d'un adhésif, **caractérisé en ce que** le morceau de matière (1) comprend une partie de base (2), une première patte (3), une seconde patte (4) et une troisième patte (5), qui sont séparées les unes des autres et partent de la partie de base sensiblement parallèlement entre elles sensiblement dans le même sens, **caractérisé en ce que** quatre sections de feuille de protection séparées (2A - 5A) sont disposées de façon amovible sur la seconde surface latérale (1") de la matière (1) pour couvrir chacune desdites parties de base et pattes (2 - 5), respectivement, où les sections de feuille sont destinées à être enlevées avant que le dispositif ne soit appliqué audit patient.

2. Dispositif selon la revendication 1, **caractérisé en ce que** la partie de base (2) est destinée à être appliquée sur la peau dudit patient à l'aide dudit adhésif.

3. Dispositif selon la revendication 2, **caractérisé en ce que** la première patte (3) est destinée à être fixée à la peau d'un côté de ladite ouverture du patient, la seconde patte intermédiaire (4) est destinée à être fixée audit tube (10) et **en ce que** la troisième patte (5) est destinée à être fixée à la peau de l'autre côté de ladite ouverture du patient.

4. Dispositif selon l'une des précédentes revendications, **caractérisé en ce qu'**au moins une des trois pattes (3 - 5) a une forme allongée.

5. Dispositif selon l'une des précédentes revendications **caractérisé en ce que** la seconde patte intermédiaire (4) est plus longue que les première et troisième pattes (3, 5).

6. Dispositif selon l'une des précédentes revendications, **caractérisé en ce que** chacune des sections de feuille (2A - 5A) comprend un volet (8) qui dépasse dans un sens opposé à la seconde surface latérale (1") de chacune des parties de base et pattes (2 - 5).

7. Dispositif selon l'une des précédentes revendications **caractérisé en ce que** la seconde surface latérale (1") a une zone d'angle (9) d'au moins une desdites parties de base et pattes (2 - 5), qui n'est pas munie dudit adhésif.

8. Dispositif selon l'une des précédentes revendications, **caractérisé en ce que** ledit élément en forme de tube comprend un tube assimilable à un flexible, qui est produit en matière plastique ou caoutchouc.

9. Dispositif selon l'une des précédentes revendications, **caractérisé en ce que** ledit élément en forme de tube comprend un tube endotrachéal.
